# EUROPEAN PATENT APPLICATION

(11) **EP 4 541 265 A1**
(43) Date of publication of application: **23.04.2025**
(21) Application number: 24207537.2
(22) Date of filing: 18.10.2024
(51) Int. Cl.: A61B 5/00, A61B 5/145

(54) **BLOOD GLUCOSE MEASUREMENT DEVICE AND METHOD FOR MANAGING BLOOD GLUCOSE MEASUREMENT DATA USING THE SAME**

(30) Priority: 19.10.2023 KR 20230140121
(71) Applicant: i-Sens, Inc., Seoul 06646 (KR)
(72) Inventor: KIM, Dong Su, 14325 Gwangmyeong-si, Gyeonggi-do (KR); KIM, Hyoung Soo, 01429 Seoul (KR); YOU, Choong Beom, 02849 Seoul (KR); LEE, Jeong Jik, 26494 Wonju-si, Gangwon-do (KR); LEE, Hye Jin, 16330 Suwon-si, Gyeonggi-do (KR)
(74) Representative: Isarpatent

(57) **Abstract**

A blood glucose measurement device according to an embodiment of the present invention comprises: a measurement unit that is partially inserted into the body of a subject and generates a blood glucose measurement signal by measuring a blood glucose concentration of the subject; a control unit that generates blood glucose measurement data by converting the blood glucose measurement signal into digital data; and a storage unit that stores the blood glucose measurement data, wherein the control unit stores the preset first verification data in the storage unit, and stores the blood glucose measurement data in the storage unit continuously to the first verification data.

## Description

### CROSS REFERENCE TO RELATED APPLICATION

The present application claims the priority of Korean Patent Application No. 10-2023-0140121 filed on 19 10, 2023, the entire contents of which is incorporated herein for all purposes by this reference.

### BACKGROUND OF THE INVENTION

### Field of the Invention

The embodiment relates to a blood glucose measurement device capable of continuously measuring a blood glucose level of a subject and a method for managing blood glucose measurement data of the blood glucose measurement device.

### Description of the Related Art

Diabetes is a chronic disease that occurs frequently in modern people, and in Korea, it affects more than 2 million people, which is 5% of the total population.

Diabetes occurs when the insulin produced by a pancreas is absolutely or relatively insufficient due to various causes such as obesity, stress, bad eating habits, and congenital heredity, so that a glucose content in the blood increases significantly by failing to correct the blood glucose balance.

Blood usually contains a certain concentration of glucose, and a tissue cell obtains energy from glucose.

However, if glucose increases more than necessary, it is not stored properly in a liver, muscle, or a fat cell, and is accumulated in the blood. As a result, a diabetic patient maintains much higher blood glucose level than normal people, and as the excessive blood glucose passes through the tissues as it is and is excreted in the urine, the glucose that is absolutely necessary for each tissue of the body becomes insufficient to cause abnormalities in each tissue of the body.

Diabetes is characterized by having almost no symptoms in the early stages. However, as the disease progresses, it causes symptoms specific to diabetes such as large beverage consumption, polyphagia, polyuria, weight loss, general malaise, itchy skin, and long-lasting wounds on hands and feet that do not heal, and as the disease progresses further, complications that progress to visual impairment, hypertension, kidney disease, stroke, periodontal disease, muscle cramps and neuralgia, gangrene, etc. are caused.

In order to diagnose such diabetes and manage it so as not to progress to complications, systematic blood glucose measurement and treatment must be performed in parallel.

Since diabetes requires constant blood glucose measurement for management, the demand for blood glucose measurement devices is steadily increasing. Various studies have confirmed that if diabetic patients strictly control their blood glucose, the occurrence of diabetic complications is significantly reduced. Accordingly, it is very important for diabetic patients to regularly measure their blood glucose level to control their blood glucose.

A blood collection-typed blood glucose meter (finger prick method) is generally used to manage blood glucose in diabetic patients. Although this blood collection-typed blood glucose meter helps diabetic patients manage blood glucose, it is difficult to accurately grasp blood glucose levels that change frequently because it only shows the results at the time of measurement. In addition, the blood collection-typed blood glucose meter requires blood to be drawn multiple times a day to measure blood glucose level, which poses a significant burden to diabetic patients.

Diabetic patients generally travel between hyperglycemia and hypoglycemia, and an emergency occurs in hypoglycemia. Hypoglycemia occurs when glucose levels do not last long, and may lead to loss of consciousness or death in the worst case.

Therefore, it is very important for diabetic patients to immediately detect hypoglycemia. However, the blood collection-typed blood glucose meter that measures blood glucose intermittently has clear limitations.

In order to overcome the limitations of the blood collection-typed blood glucose meter, a continuous glucose monitoring system (CGMS) that is inserted into the body and measures blood glucose at intervals of several minutes has been developed, and this system can be used to easily manage diabetes patients and respond to emergencies.

The continuous glucose monitoring system is configured to include a transmitter that is attached to a user's body part to measure blood glucose by extracting body fluid, and a communication terminal that outputs the transmitted blood glucose level. The sensor transmitter generates blood glucose information by measuring the user's blood glucose for a certain period of time, for example, for approximately 15 days, while the sensor is inserted into the body. The sensor transmitter periodically generates blood glucose information, and the communication terminal is installed with a blood glucose management application to periodically receive blood glucose information from the transmitter and output the received blood glucose information so that the user can check it. At this time, the transmitter stores the measured blood glucose information in a memory before generating the blood glucose information and transmitting it to the communication terminal.

However, the small embedded device such as the transmitter may experience temporary power supply problems due to external impact or electromagnetic waves. If such problems occur during storing data in a non-volatile memory of the transmitter, the data may not be stored normally. When the stored data is read to control the operation of the transmitter or transmit it to other device, if the data is not completely stored data, it may cause serious problems.

Background art of the present invention includes Japanese Patent Laid-open Publication No. 2011-062335 and U.S. Patent Application Publication No. US2010/0168537.

### SUMMARY OF THE INVENTION

The embodiment provides a blood glucose measurement device for providing accuracy and completeness to blood glucose measurement data of a subject, and a method for managing the blood glucose measurement data using the same.

The challenge which is intended to be solved by the embodiment is not limited thereto, and may also include the purpose or effect that can be identified from the solution or embodiment of the challenge to be described below.

A blood glucose measurement device according to an embodiment of the present invention comprises: a measurement unit that is partially inserted into the body of a subject and generates a blood glucose measurement signal by measuring a blood glucose concentration of the subject; a control unit that generates blood glucose measurement data by converting the blood glucose measurement signal into digital data; and a storage unit that stores the blood glucose measurement data,
wherein the control unit stores preset first verification data in the storage unit, and stores the blood glucose measurement data in the storage unit continuously to the first verification data.

If a preset predetermined condition is satisfied, the control unit may store second verification data continuously to the blood glucose measurement data.

If storage of the blood glucose measurement data is completed without the occurrence of a specific event during the storage of the blood glucose measurement data, the control unit may store the second verification data continuously to the blood glucose measurement data.

If storage of the blood glucose measurement data is stopped due to the occurrence of a specific event during the storage of the blood glucose measurement data, the control unit may store dummy data by the file size corresponding to the remaining portion of the blood glucose measurement data and the file size of the second verification data from the position where transmission was stopped.

The blood glucose measurement device may further comprise a communication unit that transmits the stored blood glucose measurement data based on the control of the control unit.

The control unit compares at least one of the first verification data and the second verification data with a specific value corresponding to each of them; if at least one of the first verification data and the second verification data and the specific value corresponding to each of them match each other, transmits the blood glucose measurement data to an external device through the communication unit; and if at least one of the first verification data and the second verification data and the specific value corresponding to each of them do not match each other, transmits flag data and the blood glucose measurement data to the external device through the communication unit, wherein the flag data may be data indicating the occurrence of an error for the blood glucose measurement data.

At least one of the first verification data and the second verification data may have a file size of at least 1 byte.

At least one of the first verification data and the second verification data may include at least one of a CRC value or a parity bit.

A method for managing blood glucose measurement data using a blood glucose measurement device that continuously measures blood glucose of a subject according to an embodiment of the present invention comprises the steps of: generating a blood glucose measurement signal by measuring blood glucose of the subject; converting the blood glucose measurement signal into blood glucose measurement data that is a digital signal; and storing the blood glucose measurement data, wherein the step of storing the blood glucose measurement data includes storing preset first verification data; and storing the blood glucose measurement data continuously to the first verification data.

The step of storing the blood glucose measurement data may further include storing second verification data continuously to the blood glucose measurement data if a preset predetermined condition is satisfied.

The step of storing the second verification data may include storing the second verification data continuously to the blood glucose measurement data if the storage of the blood glucose measurement data is completed without the occurrence of a specific event during the storage of the blood glucose measurement data.

The step of storing the second verification data may include storing dummy data by the file size corresponding to the remaining portion of the blood glucose measurement data and the file size of the second verification data from the position where transmission was stopped if the storage of the blood glucose measurement data is stopped due to the occurrence of a specific event during the storage of the blood glucose measurement data.

The method for managing blood glucose measurement data may further comprise a step of transmitting the stored blood glucose measurement data.

The step of transmitting the blood glucose measurement data includes the steps of: comparing at least one of the first verification data and the second verification data with a specific value corresponding to each of them; transmitting the blood glucose measurement data to an external device if at least one of the first verification data and the second verification data and the specific value corresponding to each of them match each other; and transmitting flag data and the blood glucose measurement data to the external device if at least one of the first verification data and the second verification data and the specific value corresponding to each of them do not match each other,
wherein the flag data may be data indicating the occurrence of an error for the blood glucose measurement data.

At least one of the first verification data and the second verification data may have a file size of at least 1 byte.

At least one of the first verification data and the second verification data may include at least one of a CRC value or a parity bit.

According to an embodiment, the present invention can provide information on accuracy, completeness, and validity together in using blood glucose measurement data later, by storing verification data together so that the validity of blood glucose measurement data can be checked when the transmitter stores the blood glucose measurement data. Accordingly, the user can detect inaccurate, incomplete, and invalid data, thereby providing an advantage capable of trusting and using the blood glucose measurement data.

The present invention can transmit error information on incorrectly stored data together with the blood glucose measurement data, thereby providing an advantage of allowing a user terminal that receives the blood glucose measurement data to perform appropriate error processing therefor.

The various and beneficial advantages and effects of the present invention are not limited to the above-described contents, and will be able to be more easily understood in the course of disclosing specific embodiments of the present invention.

### BRIEF DESCRIPTION OF THE DRAWINGS

FIG. 1 is a schematic diagram illustrating a continuous glucose monitoring system according to an embodiment of the present invention.
FIG. 2 is a diagram for explaining configurations of a blood glucose measurement device according to an embodiment of the present invention.
FIG. 3 is a diagram for explaining a storage process of blood glucose measurement data according to a first embodiment of the present invention.
FIG. 4 is a diagram for explaining a storage process of blood glucose measurement data according to a second embodiment of the present invention.
FIG. 5 is a diagram for explaining a storage process of blood glucose measurement data according to a third embodiment of the present invention.
FIG. 6 is a diagram for explaining a storage process of blood glucose measurement data according to a fourth embodiment of the present invention.
FIG. 7 is a diagram for explaining a storage process of blood glucose measurement data according to a fifth embodiment of the present invention.
FIG. 8 is a diagram for explaining a storage process of blood glucose measurement data according to a sixth embodiment of the present invention.
FIG. 9 is a diagram for explaining a data verification process using first verification data and second verification data according to an embodiment of the present invention.
FIG. 10 is a flowchart illustrating a method for managing blood glucose measurement data according to an embodiment of the present invention.
FIG. 11 is a diagram illustrating an embodiment of step S1030 of FIG. 10 in detail.
FIG. 12 is a diagram illustrating an embodiment of step S1030 of FIG. 10 in detail.
FIG. 13 is a diagram illustrating an embodiment of step S1040 of FIG. 10 in detail.

### DETAILED DESCRIPTION OF THE INVENTION

Since the present invention may make various modifications and have various embodiments, specific embodiments will be illustrated in the drawings and described in detail. However, this is not intended to limit the present invention to the specific embodiments, and it should be understood that this includes all modifications, equivalents, or substitutes included in the spirit and technical scope of the present invention.

The terms such that include ordinal numbers, such as second and first may be used to describe various components, but the components should not be limited by these terms. The terms are used only for the purpose of distinguishing one component from another component. For example, without departing from the scope of the present invention, a second component may be referred to as a first component, and likewise, the first component may also be referred to as the second component. The term "and/or" includes a combination of a plurality of related/described items or any one of the plurality of related/described items.

In case a certain component is referred to as being "linked" or "connected" to another component, it should be understood that there may be other component between them, although the certain component may be directly linked to or connected to another component. On the other hand, in case a certain component is referred to as being "directly linked" or "directly connected" to another component, it should be understood that there are no other component between them.

The terms used in the present application are used merely to describe specific embodiments and are not intended to limit the present invention. A singular expression covers a plural expression unless the context clearly indicates otherwise. In the present application, the terms such as "comprise" or "have" are intended to designate the presence of a feature, number, step, operation, component, part, or combination thereof described in the specification, but should be understood not to preclude the presence or additional possibility of one or more other features, numbers, steps, operations, components, parts, or combinations thereof.

Unless defined otherwise, all terms used herein, including technical or scientific terms, have the same meaning as generally understood by a person who has an ordinary knowledge in the technical field to which the present invention belongs. The terms such as those generally defined in the dictionary should be interpreted as having a meaning consistent with the meaning of the context of the relevant technology, and shall not be interpreted in an ideal or overly formal sense unless defined in the present application explicitly.

Hereinafter, with reference to the attached drawings, embodiments will be described in detail. Regardless of the drawing symbol, the same or corresponding components are assigned to the same reference number, and duplicate descriptions thereof will be omitted.

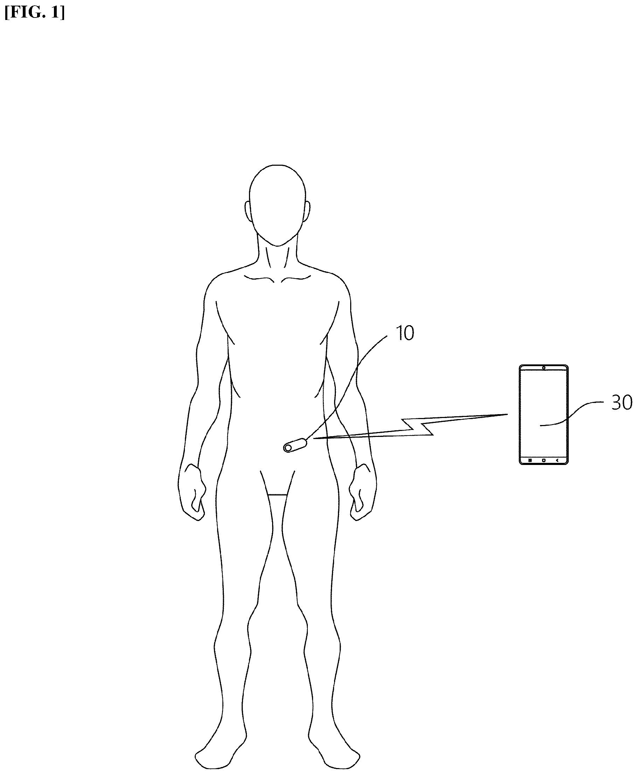

FIG. 1 is a schematic diagram illustrating a continuous glucose monitoring system according to an embodiment of the present invention.

Referring to FIG. 1, the continuous glucose monitoring system according to an embodiment of the present invention comprises a blood glucose measurement device 10 and a user terminal 30.

The blood glucose measurement device 10 is attached to the body of a user, i.e., a subject. A part of the blood glucose measurement device 10 may be inserted into the skin when attached to the body. The blood glucose measurement device 10 having the part inserted into the skin may measure blood glucose by periodically extracting body fluid. In an embodiment, the body fluid may be interstitial fluid. The blood glucose measurement device 10 may be expressed as a transmitter.

The user terminal 30 receives blood glucose information from the blood glucose measurement device 10. The user terminal 30 may mean a terminal device that can display the received blood glucose information to the user. The user terminal 30 may include a mobile terminal device that can communicate with the blood glucose measurement device 10, such as a smart phone, a tablet PC, or a notebook computer. However, the user terminal 30 is not limited thereto, and any terminal device that includes a communication function and is capable of installing a program or application may be included in the user terminal 30. The user terminal 30 may be used to mean a terminal used by a third party in addition to the terminal used by the subject. The user terminal 30 may be expressed as an external device.

The blood glucose measurement device 10 may transmit blood glucose information measured periodically at the request of the user terminal 30 or at preset every time to the user terminal 30. In this case, the blood glucose measurement device 10 may be connected to the user terminal 30 in a wired communication manner such as a USB cable for data communication, or may be connected to the user terminal 30 in a wireless communication manner such as infrared communication, NFC (Near Field Communication), Bluetooth, WiFi (Wireless Fidelity), LTE, etc.

The blood glucose measurement device 10 may convert a blood glucose measurement signal into blood glucose measurement data in order to transmit blood glucose information to the user terminal 30. The blood glucose measurement signal may be an analog signal measured by a sensor, and the blood glucose measurement data may be a digital signal.

Further, the blood glucose measurement device 10 may store the blood glucose measurement data in order to transmit blood glucose information to the user terminal 30. The blood glucose measurement device 10 may transmit the blood glucose measurement data to the user terminal 30 based on a request from the user terminal 30 or a predetermined transmission rule after storing the blood glucose measurement data.

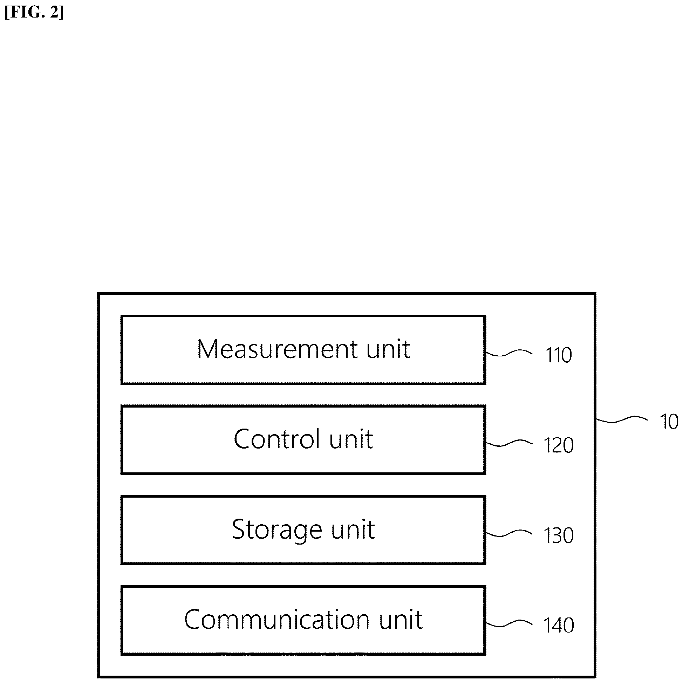

FIG. 2 is a diagram for explaining configurations of a blood glucose measurement device according to an embodiment of the present invention.

Referring to FIG. 2, the blood glucose measurement device according to an embodiment of the present invention, i.e., the transmitter, may comprise a measurement unit 110, a control unit 120, a storage unit 130, and a communication unit 140.

First, the measurement unit 110 measures a biometric signal of a subject. The biometric signal may include a blood glucose measurement signal. The measurement unit 110 may be inserted into a part of the body of the subject to measure a blood glucose concentration of the subject and generate the blood glucose measurement signal. One end of the measurement unit 110 may be inserted into a skin of the subject to extract body fluid and measure blood glucose. A part of the measurement unit 110 inserted into a part of the subject's body may be a needle.

The measurement unit 110 may periodically extract body fluid and measure blood glucose. For example, the measurement unit 110 may extract body fluid at time intervals of 10 seconds and measure blood glucose. The measurement unit 110 may measure a plurality of blood glucose levels per one cycle of extracting body fluid. For example, the measurement unit 110 may measure blood glucose levels 30 times for 1 second per one cycle. The measurement unit 110 may periodically extract body fluid and measure a plurality of blood glucose levels per one cycle. For example, the measurement unit 110 may measure blood glucose 30 times between 14 minutes 00 seconds and 14 minutes 01 seconds, measure blood glucose 30 times between 14 minutes 11 seconds and 14 minutes 12 seconds, and measure blood glucose 30 times between 14 minutes 22 seconds and 14 minutes 23 seconds.

Next, the control unit 120 may convert the biometric signal, which is an analog signal, into biometric data, which is a digital signal. The control unit 120 may convert the blood glucose measurement signal into digital data to generate blood glucose measurement data.

Next, the control unit 120 may control the overall operation of the transmitter. The control unit 120 may control the operation of the measurement unit 110, the communication unit 140, and the storage unit 130.

The control unit 120 may control the blood glucose measurement operation of the measurement unit 110. The control unit 120 may control intervals at which the measurement unit 110 extracts blood glucose, the number of times blood glucose is extracted per a cycle, etc.

The control unit 120 may control the storage unit 130 to store the biometric data. The control unit 120 may control the storage unit 130 to store the blood glucose measurement data.

The control unit 120 may control first verification data to be stored in the storage unit 130. The control unit 120 may control to store the blood glucose measurement data in the storage unit 130 continuously to the first verification data. If a preset predetermined condition is satisfied, the control unit 120 may control to store second verification data continuously to the blood glucose measurement data. Specifically, if the storage of blood glucose measurement data is completed without occurrence of a specific event during the storage of the blood glucose measurement data, the control unit 120 may control to store the second verification data continuously to the blood glucose measurement data. Herein, the specific event may include a case where data storage is stopped during the storage of the blood glucose measurement data. Meanwhile, if the storage of the blood glucose measurement data is stopped due to occurrence of the specific event for the storage of the blood glucose measurement data, the control unit 120 may control to store dummy data equal to s file size corresponding to the remaining portion of the blood glucose measurement data and a file size of the second verification data from a position where transmission was stopped.

The control unit 120 may control the operation of the communication unit 140 to transmit biometric data to an external device.

The control unit 120 may compare at least one of the first verification data and the second verification data with a specific value corresponding to each of them. Then, if at least one of the first verification data and the second verification data and the specific value corresponding to each of them match each other, the control unit 120 may control the communication unit 140 to transmit the blood glucose measurement data stored in the storage unit 130 to an external device via the communication unit 140.

On the other hand, if at least one of the first verification data and the second verification data, and the specific value corresponding to each of them are not consistent with each other, the storage unit 130 may control the communication unit 140 to transmit flag data and the blood glucose measurement data to an external device via the communication unit 140. Herein, the flag data may be data indicating the occurrence of an error for the blood glucose measurement data. In other words, the flag data may include an error occurrence message.

Meanwhile, at least one of the first verification data and the second verification data may have a file size of at least 1 byte. Accordingly, the first verification data and the second verification data may have various file sizes such as 1 byte, 2 bytes, and 3 bytes, which may be designed and changed by a person skilled in the art.

Further, at least one of the first verification data and the second verification data may include at least one of a CRC value or a parity bit. The CRC value or the parity bit included in at least one of the first verification data and the second verification data may be used to detect an error in the data.

Furthermore, the first verification data and the second verification data may have the same value, but is not limited thereto. The first verification data and the second verification data may have different values from each other.

Next, the storage unit 130 may store biometric data. The storage unit 130 may store the blood glucose measurement data. The storage unit 130 may store the blood glucose measurement data depending on the control of the control unit 120.

The storage unit 130 may include a memory capable of storing data. According to an embodiment, the storage unit 130 may be a non-volatile memory, but is not limited thereto.

Then, the communication unit 140 may transmit biometric data to an external device. The communication unit 140 may transmit the blood glucose measurement data to the external device.

The communication unit 140 may include an infrared communication module, an NFC (Near Field Communication) module, a Bluetooth module, a WiFi (Wireless Fidelity) module, an LTE module, etc.

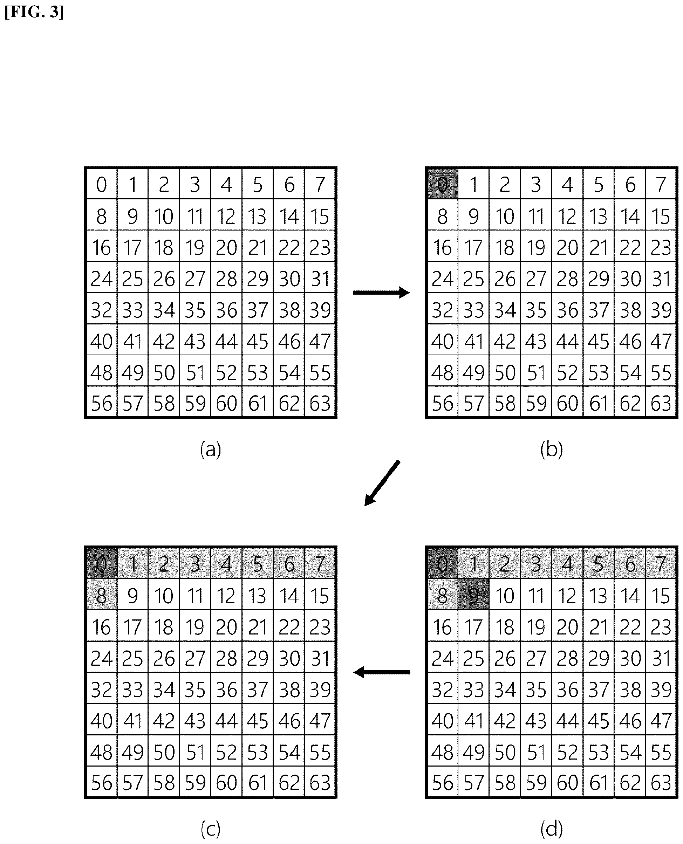

FIG. 3 is a diagram for explaining a storage process of blood glucose measurement data according to a first embodiment of the present invention.

FIG. 3(a) is a diagram schematically illustrating a memory used for storing data in rows and columns. FIG. 3(a) shows a memory schematically illustrated in eight rows and eight columns. In FIG. 3(a), each cell represents a size of 1 byte, and therefore, the memory illustrated in the diagram represents a memory having a storage capacity of 64 bytes. The expression "memory" in the description may be used interchangeably with the storage unit 130 described above. In addition, the expression "memory" in the description may be used interchangeably with a term indicating a storage space unit of the memory. For example, the matrix schematically illustrating the memory in FIG. 3(a) may mean a sector for data storage. Herein, the sector may include both a physical sector and a logical sector. The matrix of FIG. 3(a) represents a state in which no data is currently stored.

The control unit 120 converts the blood glucose measurement signal measured by a sensor unit into digital data, i.e., blood glucose measurement data, and then stores the blood glucose measurement data in the memory illustrated in FIG. 3(a).

To this end, first, as illustrated in FIG. 3(b), the control unit 120 may store first verification data having a size of 1 byte in the storage space of index 0 of the memory. According to an embodiment, the first verification data may be data including the hexadecimal number "0x53". According to other embodiment, the first verification data may be data including the hexadecimal number "0x43". According to another embodiment, the first verification data may be data including the hexadecimal number "0x45". In the diagram, the size of the first verification data is expressed as 1 byte, but is not limited thereto. According to an embodiment of the present invention, the first verification data may have a size of 2 bytes. Further, the first verification data may have a size of 3 bytes. Furthermore, the first verification data may have a size of 4 bytes. The size of the first verification data may be changed to a size of 1 byte or more by a person skilled in the art.

Next, as shown in FIG. 3(c), the control unit 120 may store blood glucose measurement data having a size of 8 bytes from the storage space of the 1^{st} index to the storage space of the 8^{th} index continuously to the first verification data. Herein, the blood glucose measurement data is indicated as 8 bytes for explanation purposes and the size is not limited thereto. The size of the blood glucose measurement data may be changed by a person skilled in the art depending on various factors such as the specification of the blood glucose measurement device, the usage environment, etc.

Next, as shown in FIG. 3(d), the control unit 120 may store second verification data having a size of 1 byte in the storage space of the 9^{th} index continuously to the blood glucose measurement data. According to an embodiment, the second verification data may be data including the hexadecimal number "0x53". According to other embodiment, the second verification data may be data including the hexadecimal number "0x43". According to another embodiment, the second verification data may be data including the hexadecimal number "0x45". In the diagram, the size of the second verification data is expressed as 1 byte, but the size is not limited thereto. According to an embodiment of the present invention, the second verification data may have a size of 2 bytes. Further, the second verification data may have a size of 3 bytes. Furthermore, the second verification data may have a size of 4 bytes. The size of the second verification data may be changed to a size of 1 byte or more by a person skilled in the art.

As such, the blood glucose measurement data may be stored in the memory by arranging it between the first verification data and the second verification data.

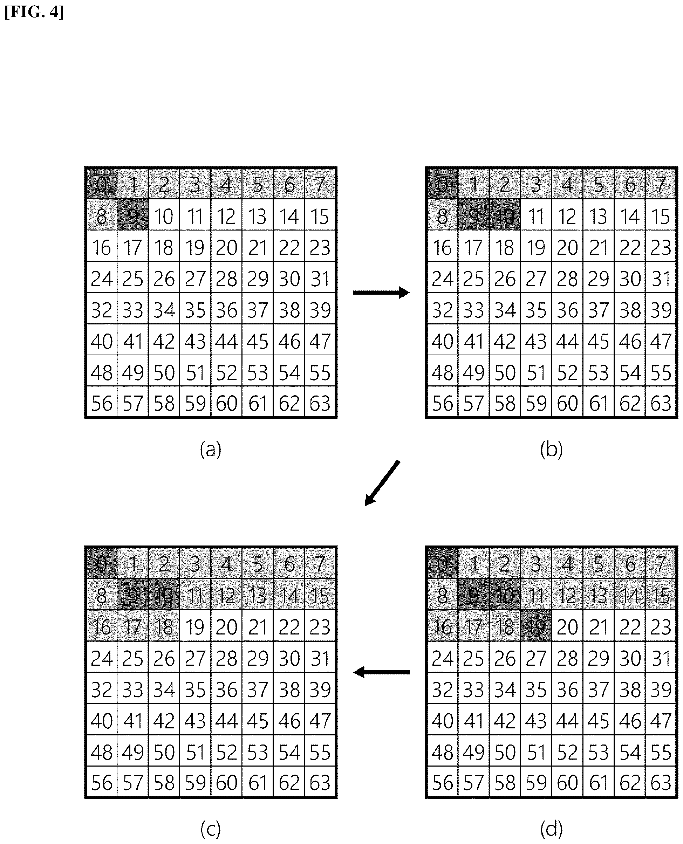

FIG. 4 is a diagram for explaining a process of storing blood glucose measurement data according to a second embodiment of the present invention.

FIG. 4 shows a process of storing a plurality of blood glucose measurement data. The following describes an embodiment with reference to FIG. 4, and therefore the description of parts that overlap with the previously explained contents will be omitted.

The diagram illustrated in FIG. 4 shows a process of storing second blood glucose measurement data after first blood glucose measurement data is stored. Herein, the first blood glucose measurement data and the second blood glucose measurement data may mean information measuring blood glucose of a subject at different points of time. For example, it is assumed that the blood glucose of the subject is measured 30 times per second and the measurement are repeated at intervals of 10 seconds. In other words, there may be first blood glucose measurements of 30 times between 0 and 1 second, and second blood glucose measurements of 30 between 11 and 12 seconds, which is 10 seconds later. In this case, as an example, the first blood glucose measurement data may be data regarding the blood glucose measurements of the first 30 times, and the second blood glucose measurement data may be data regarding the blood glucose measurements of the second 30 times. Also, as another example, the first blood glucose measurement data may be data regarding blood glucose measurement of one time among the blood glucose measurements of the first 30 times, and the second blood glucose measurement data may be data regarding blood glucose measurements of 2 times among the blood glucose measurements of the first 30 times.

The control unit 120 converts a first blood glucose measurement signal and a second blood glucose measurement signal measured by a sensor unit into digital data, i.e., the first blood glucose measurement data and the second blood glucose measurement data, respectively, and then stores the blood glucose measurement data in the memory.

First, referring to FIG. 4(a), the control unit 120 stores the first blood glucose measurement data. The control unit 120 stores first verification data of 1 byte size in the storage space of the 0^{th} index, stores the first blood glucose measurement data in the storage space from the 2^{nd} index to the 8^{th} index, and stores second verification data of 1 byte size in the storage space of the 9^{th} index.

Next, as illustrated in FIG. 4(b), the control unit 120 may store the first verification data of 1 byte size in the storage space of the 10^{th} index of the memory.

Next, as shown in FIG. 4(c), the control unit 120 may store the blood glucose measurement data having a size of 8 bytes in the storage space from the 11^{th} index to the storage space of the 18^{th} index continuously to the first verification data.

Next, as shown in FIG. 4(d), the control unit 120 may store the second verification data of 1 byte size in the storage space of the 19^{th} index continuously to the blood glucose measurement data.

Herein, in order to provide convenience in understanding the invention, although the first blood glucose measurement data and the second blood glucose measurement data are described as examples, they are not limited thereto. A plurality of blood glucose measurement data such as third blood glucose measurement data may also be stored in the storage unit 130 depending on the control of the control unit 120 in the same manner as described above.

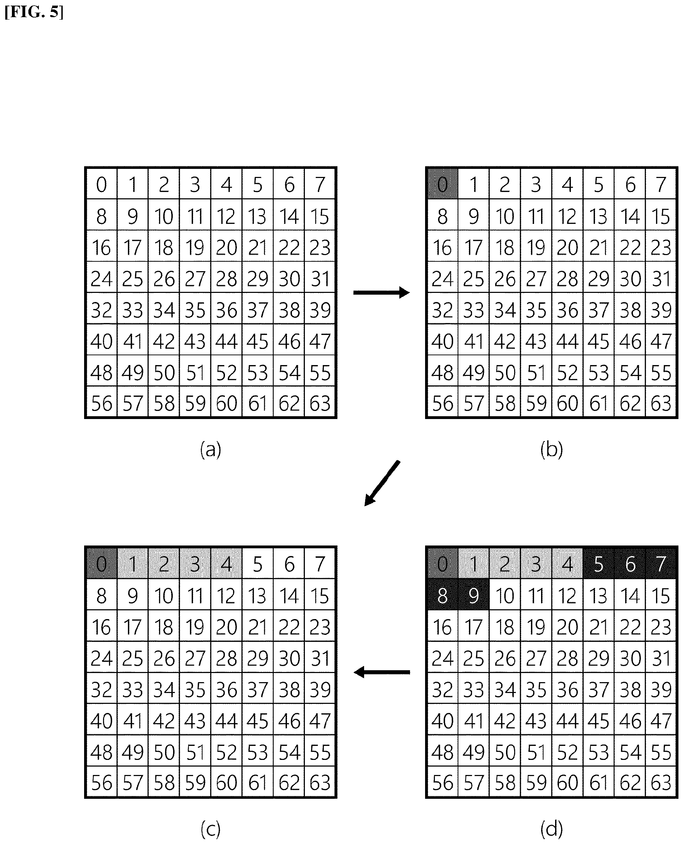

FIG. 5 is a diagram for explaining a storage process of blood glucose measurement data according to a third embodiment of the present invention.

FIG. 5 is presented with the assumption that, for example, data storage is stopped due to the occurrence of a specific event during storage of the blood glucose measurement data. Hereinafter, an embodiment will be described with reference to FIG. 5, but the description of parts that overlap with the previously described contents will be omitted.

The data storage process illustrated in FIG. 5 describes that, for example, a specific event occurs and data storage is stopped during storage of the blood glucose measurement data. The specific event may include a case where the power supply is temporarily interrupted due to an external electromagnetic wave, a case where the power supply to a battery is temporarily interrupted due to an external impact, etc. In addition, the specific event may include various events that contribute to interrupting the data storage process.

The control unit 120 converts a blood glucose measurement signal measured by a sensor unit into digital data, i.e. blood glucose measurement data, and then stores the blood glucose measurement data in the memory illustrated in FIG. 5(a).

To this end, first, as illustrated in FIG. 5(b), the control unit 120 may store first verification data of 1 byte size in the storage space of the 0^{th} index of the memory.

Next, as shown in FIG. 5(c), the control unit 120 may store the blood glucose measurement data starting from the storage space of the 1^{st} index of the memory. During sequential storage of the blood glucose measurement data starting from the storage space of the 1^{st} index of the memory, if a specific event occurs and data storage is stopped after storing the blood glucose measurement data in the storage space of the 4^{th} index, the control unit 120 does not store the blood glucose measurement data from the storage space of the 5^{th} index to the storage space of the 8^{th} index.

Next, as shown in FIG. 5(d), the control unit 120 stores dummy data from the storage space of the 5^{th} index to the storage space of the 9^{th} index. The storage spaces from the 5^{th} index to the 8^{th} index is a storage space allocated for storing the blood glucose measurement data, and is a space where the blood glucose measurement data is not stored due to storage stop of the blood glucose measurement data. In addition, the storage space of the 9^{th} index is a space allocated for storing second verification data.

As such, if data storage is stopped during storage of the blood glucose measurement data, the control unit 120 stores dummy data in the storage space where the blood glucose measurement data fails to be stored and the storage space where the second verification data should be stored after the stop. In other words, a data value of the dummy data may be stored instead of the data value that should be stored as the second verification data.

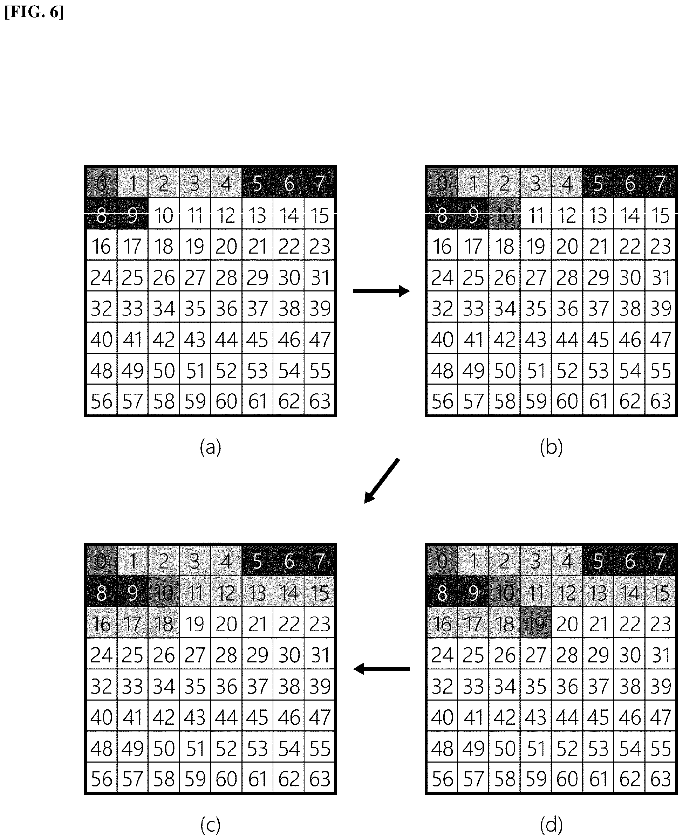

FIG. 6 is a diagram for explaining a storage process of blood glucose measurement data according to a fourth embodiment of the present invention.

FIG. 6 shows the process of storing a plurality of blood glucose measurement data. In particular, FIG. 6 is presented on the assumption that a specific event occurs during storage of first blood glucose measurement data and does not occur during storage of second blood glucose measurement data. The following describes an embodiment with reference to FIG. 6, and therefore the description of parts that overlap with the previously described contents will be omitted.

The control unit 120 converts a first blood glucose measurement signal and a second blood glucose measurement signal measured by a sensor unit into digital data, i.e., first blood glucose measurement data and second blood glucose measurement data, respectively, and then stores the blood glucose measurement data in a memory.

First, FIG. 6(a) shows a state in which the first blood glucose measurement data is stored when a specific event occurs as described in FIG. 5 and data storage is stopped during storage of the blood glucose measurement data. The control unit 120 stores first verification data of 1 byte size in the storage space of the 0^{th} index, stores the first blood glucose measurement data in the storage spaces from the 2^{nd} index to the 4^{th} index, and stores dummy data in the storage spaces from the 5^{th} index to the 9^{th} index.

Next, as shown in FIG. 6(b), the control unit 120 may store the first verification data of 1 byte size in the storage space of the 10^{th} index of the memory.

Next, as shown in FIG. 6(c), the control unit 120 may store the blood glucose measurement data having a size of 8 bytes from the storage space of the 11^{th} index to the storage space of the 18^{th} index continuously to the first verification data.

Next, as shown in FIG. 6(d), the control unit 120 may store the second verification data of 1 byte size in the storage space of the 19^{th} index continuously to the blood glucose measurement data.

Herein, in order to provide convenience in understanding the invention, the first blood glucose measurement data and the second blood glucose measurement data are described as examples, but the invention is not limited thereto. A plurality of blood glucose measurement data such as third blood glucose measurement data may also be stored in the storage unit 130 depending on the control of the control unit 120 in the same manner as described above.

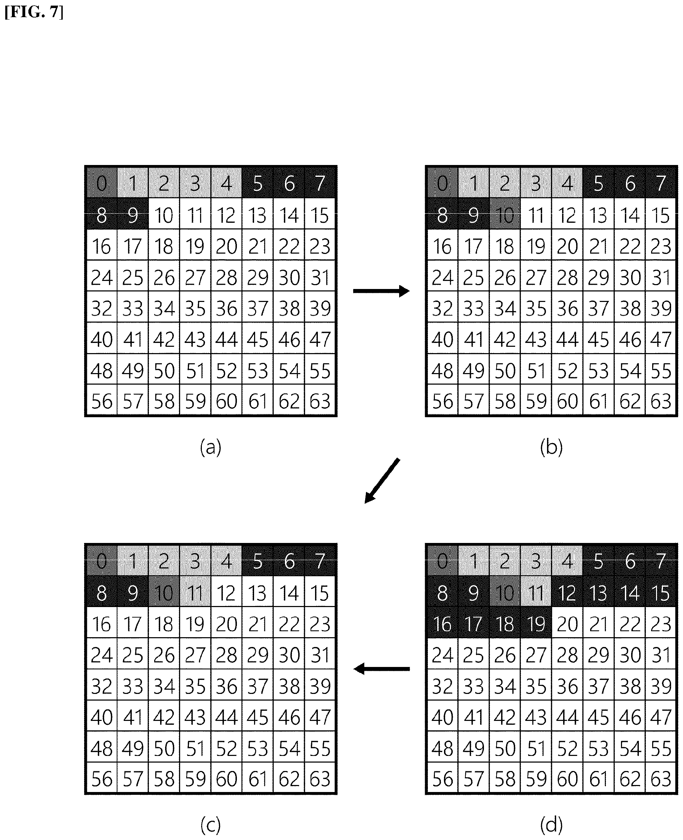

FIG. 7 is a diagram for explaining a storage process of blood glucose measurement data according to a fifth embodiment of the present invention.

FIG. 7 shows a process of storing a plurality of blood glucose measurement data. In particular, FIG. 7 is presented on the assumption that a specific event occurs during storage of first blood glucose measurement data and occurs also during storage of second blood glucose measurement data. Hereinafter, an embodiment will be described with reference to FIG. 7, but the explanation of parts that overlap with the previously described contents will be omitted.

The control unit 120 converts a first blood glucose measurement signal and a second blood glucose measurement signal measured by a sensor unit into digital data, i.e., first blood glucose measurement data and second blood glucose measurement data, respectively, and then stores the blood glucose measurement data in a memory.

First, FIG. 7(a) shows a state in which the first blood glucose measurement data is stored when a specific event occurs as described in FIG. 5 and data storage is stopped during storage of the blood glucose measurement data. The control unit 120 stores first verification data of 1 byte size in the storage space of the 0^{th} index, stores the first blood glucose measurement data in the storage spaces from the 2^{nd} index to the 4^{th} index, and stores dummy data in the storage spaces from the 5^{th} index to 9^{th} index.

Next, as shown in FIG. 7(b), the control unit 120 may store the first verification data of 1 byte size in the storage space of the 10^{th} index of a memory.

Next, as shown in FIG. 7(c), the control unit 120 may store the blood glucose measurement data having a size of 8 bytes in the storage space of the 11^{th} index continuously to the first verification data. During sequential storage of the blood glucose measurement data in the storage space of the 11^{th} index of the memory, if a specific event occurs and data storage is stopped after storing the blood glucose measurement data in the storage space of the 11^{th} index, the control unit 120 does not store the blood glucose measurement data in the storage spaces from the 12^{th} index to the 18^{th} index.

Next, as shown in FIG. 7(d), the control unit 120 stores dummy data in the storage spaces from the 12^{th} index to the 19^{th} index. The storage spaces from the 12^{th} index to the 18^{th} index are a storage space allocated for storing the blood glucose measurement data, and is a space where the blood glucose measurement data is not stored due to storage stop of the blood glucose measurement data. In addition, the storage space of the 19^{th} index is a space allocated for storing second verification data.

Herein, in order to provide convenience in understanding the invention, the first blood glucose measurement data and the second blood glucose measurement data are described as examples, but they are not limited thereto. A plurality of blood glucose measurement data such as third blood glucose measurement data may also be stored in the storage unit 130 depending on the control of the control unit 120 in the same manner as described above.

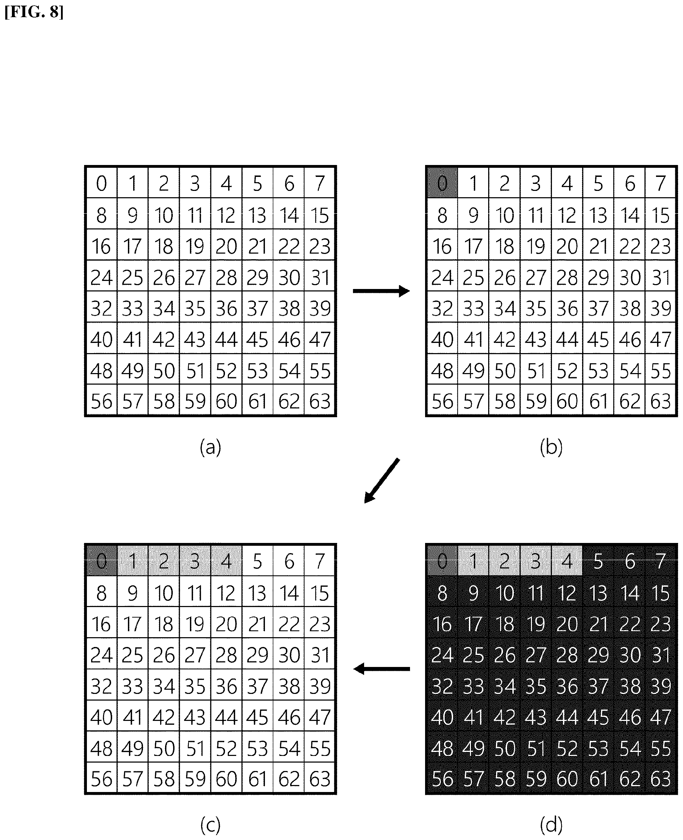

FIG. 8 is a diagram for explaining a storage process of blood glucose measurement data according to a sixth embodiment of the present invention.

FIG. 8 is presented in the assumption of an example in which data storage is stopped due to the occurrence of a specific event during storage of the blood glucose measurement data. Hereinafter, an embodiment will be described with reference to FIG. 8, but the description of parts that overlap with the previously described contents will be omitted.

The control unit 120 converts a blood glucose measurement signal measured by a sensor unit into digital data, i.e., the blood glucose measurement data, and then stores the blood glucose measurement data in a memory illustrated in FIG. 8(a).

To this end, first, as illustrated in FIG. 8(b), the control unit 120 may store first verification data of 1 byte size in the storage space of the 0^{th} index of a memory.

Next, as illustrated in FIG. 8(c), the control unit 120 may store the blood glucose measurement data starting from the storage space of the 1^{st} index of the memory. In the course of sequentially storing the blood glucose measurement data starting from the storage space of the 1^{st} index of the memory, if a specific event occurs and data storage is stopped after storing the blood glucose measurement data in the storage space of the 4^{th} index, the control unit 120 does not store the blood glucose measurement data in the storage space from the 5^{th} index to the storage space of the 8^{th} index.

Next, as shown in FIG. 8(d), the control unit 120 stores dummy data in the storage spaces from the 5^{th} index to the 63^{rd} index. That is, all remaining storage spaces for the corresponding sector can be recorded as the dummy data.

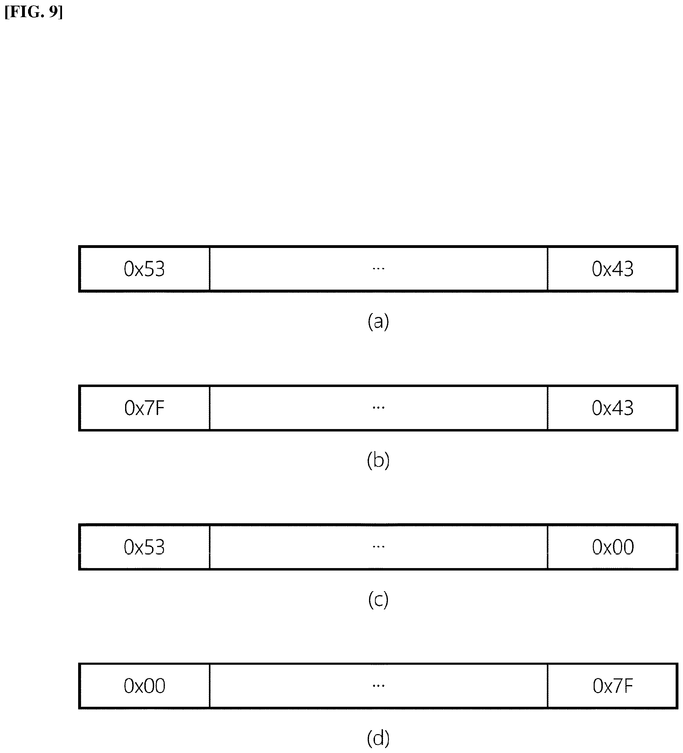

FIG. 9 is a diagram for explaining a data verification process using first verification data and second verification data according to an embodiment of the present invention.

FIG. 9 shows examples of four types that may occur when storing blood glucose data. Herein, the data value of 1 byte described in FIG. 9 is an example and the invention is not limited thereto.

Table 1 below shows specific values for comparing the first verification data and the second verification data with each other.

**[Table 1]**

| Classification | Value |
|---|---|
| First specific value | 0x53 |
| Second specific value | 0x43 |

In Table 1, the first specific value is a value for comparing with the first verification data, and the second specific value is a value for comparing with the second verification data.

The control unit 120 may verify data validity of the blood glucose measurement data before transmitting the blood glucose measurement data to an external device. In this case, the control unit 120 may verify the data validity by comparing the first verification data and the second verification data, which are stored together before and after the blood glucose measurement data during the storage process of the blood glucose measurement data, with the first specific value and the second specific value, respectively.

First, in FIG. 9(a), [0x53] is stored as the value of the first verification data, and [0x43] is stored as the value of the second verification data. In this case, [0x53], which is the value of the first verification data, matches with [0x53], which is the first specific value, and [0x43], which is the value of the second verification data, matches with [0x43], which is the second specific value. That is, since both the first verification data and the second verification data match the preset specific values, the control unit 120 can determine that the corresponding blood glucose measurement data is valid, and therefore, the control unit 120 can transmit the corresponding blood glucose measurement data to an external device.

Next, in FIG. 9(b), [0x7F] is stored as the value of the first verification data, and [0x43] is stored as the value of the second verification data. In this case, [0x7F], the value of the first verification data, does not match with [0x53], the first specific value. On the other hand, [0x43], the value of the second verification data, matches with [0x43], the second specific value. That is, since only the second verification data among the first verification data and the second verification data matches the preset specific value, the control unit 120 can determine that the corresponding blood glucose measurement data is invalid, and therefore, the blood glucose measurement data and the flag data can be transmitted to an external device.

Next, in FIG. 9(c), [0x53] is stored as the value of the first verification data, and [0x7F] is stored as the value of the second verification data. In this case, [0x53], the value of the first verification data, matches with [0x53], the first specific value. On the other hand, [0x00], the value of the second verification data, does not match with [0x43], the second specific value. That is, since only the first verification data among the first verification data and the second verification data matches all the preset specific values, the control unit 120 can determine that the corresponding blood glucose measurement data is invalid, and therefore, the blood glucose measurement data and the flag data can be transmitted to an external device.

Next, in FIG. 9(d), [0x00] is stored as the value of the first verification data, and [0x7F] is stored as the value of the second verification data. In this case, [0x00], the value of the first verification data, does not match with [0x53], the first specific value. In addition, [0x7F] of the second verification data does not match with [0x43], the second specific value. That is, since both the first verification data and the second verification data do not match with the preset specific values, the control unit 120 can determine that the corresponding blood glucose measurement data is invalid, and therefore, the blood glucose measurement data and the flag data can be transmitted to an external device.

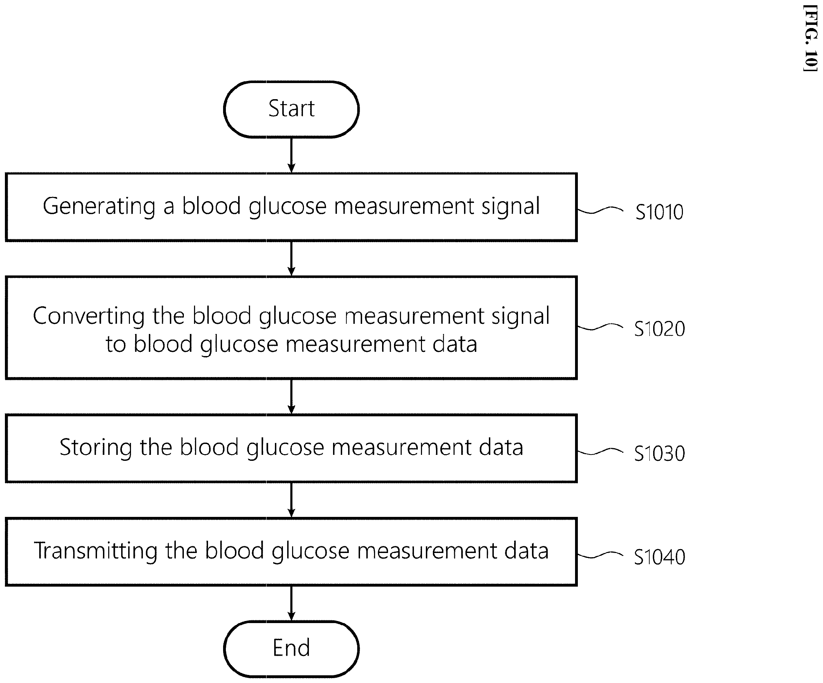

FIG. 10 is a flowchart illustrating a method for managing blood glucose measurement data according to an embodiment of the present invention.

The method for managing blood glucose measurement data as shown in FIG. 10 may be performed using the blood glucose measurement device 10, i.e., the transmitter, as described above.

Referring to FIG. 10, first, the measurement unit 110 generates a blood glucose measurement signal by measuring blood glucose of a subject (S1010).

Next, the control unit 120 converts the blood glucose measurement signal into blood glucose measurement data, i.e., a digital signal (S1020).

Next, the storage unit 130 stores the blood glucose measurement data (S1030). The storage of the blood glucose measurement data may be controlled by the control unit 120.

Next, the communication unit 140 transmits the stored blood glucose measurement data to an external device (S1040). The transmission of the blood glucose measurement data may be controlled by the control unit 120.

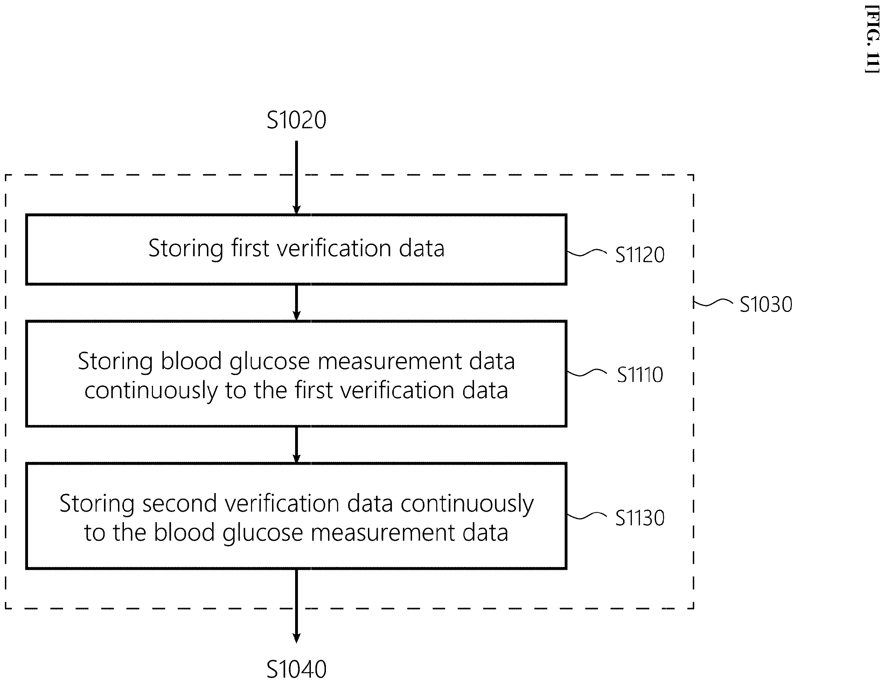

FIG. 11 is a diagram illustrating an embodiment of step S1030 of FIG. 10 in detail.

Referring to FIG. 11, the control unit 120 may control to store preset first verification data in the storage unit 130 (S1110). That is, the storage unit 130 may store the preset first verification data.

Also, the control unit 120 may control to store the blood glucose measurement data in the storage unit 130 continuously to the first verification data (S1120). That is, the storage unit 130 may store the blood glucose measurement data continuously to the first verification data.

Next, the control unit 120 may control to store second verification data in the storage unit 130 continuously to the blood glucose measurement data if a preset predetermined condition is satisfied (S1130). That is, the storage unit 130 may store the second verification data continuously to the blood glucose measurement data if the preset predetermined condition is satisfied.

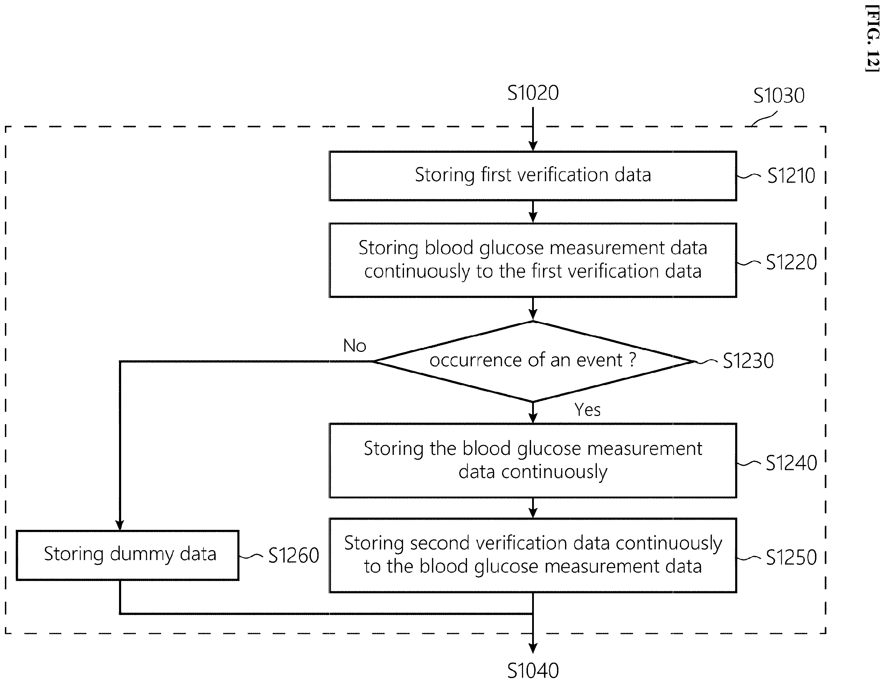

FIG. 12 is a diagram illustrating an embodiment of step S1030 of FIG. 10 in detail.

Referring to FIG. 12, the control unit 120 may control to store preset first verification data in the storage unit 130 (S1110). That is, the storage unit 130 may store the preset first verification data.

Also, the control unit 120 may control to store the blood glucose measurement data in the storage unit 130 continuously to first verification data (S1120). That is, the storage unit 130 may store the blood glucose measurement data continuously to the first verification data.

Next, the control unit 120 may determine whether a specific event has occurred (S1230).

If storage of the blood glucose measurement data is completed without the occurrence of a specific event during the storage of the blood glucose measurement data, the control unit 120 may control to continuously store the blood glucose measurement data (S1240) and to store second verification data in the storage unit 130 continuously to the blood glucose measurement data (S1250). That is, the storage unit 130 may continuously store the blood glucose measurement data and then continuously store the second verification data.

Meanwhile, if the storage of blood glucose measurement data is stopped due to the occurrence of a specific event in the course of storing the blood glucose measurement data, the control unit 120 may control to store dummy data in the storage unit 130 by the file size corresponding to the remaining portion of the blood glucose measurement data and the file size of the second verification data from the position where transmission was stopped (S 1260). That is, the storage unit 130 may store the dummy data in the storage space allocated to the blood glucose measurement data and the storage space allocated to the second verification data from the position where the data transmission was stopped.

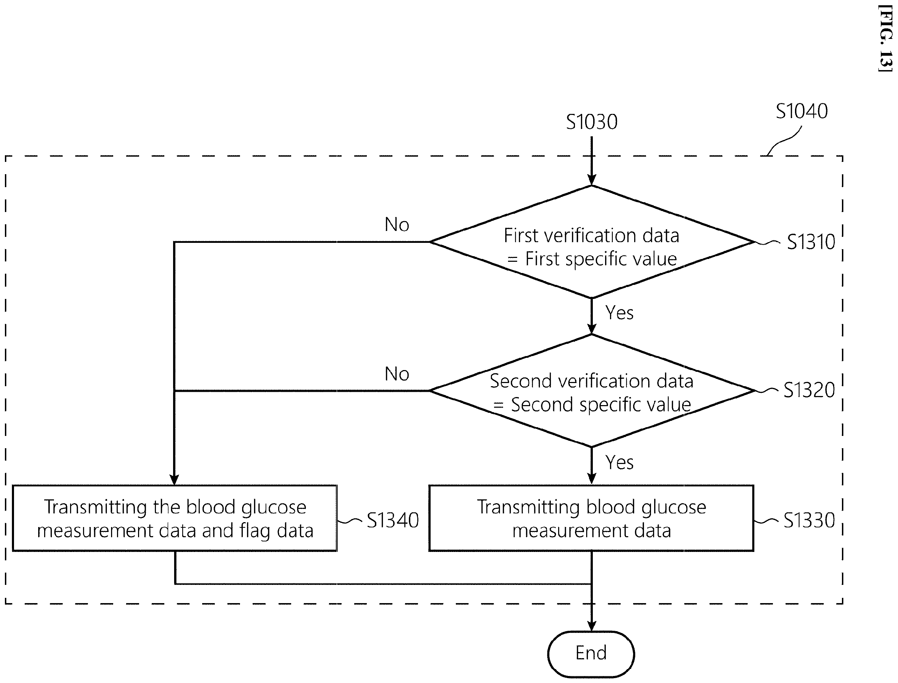

FIG. 13 is a diagram illustrating an embodiment of step S1040 of FIG. 10 in detail.

Referring to FIG. 13, the control unit 120 may compare first verification data with a first specific value (S1310).

If the first verification data and the first specific value match each other, the control unit 120 may compare second verification data with a second specific value (S1320).

If the second verification data and the second specific value match each with each other, the control unit 120 may transmit the blood glucose measurement data to an external device (S1330). That is, the communication unit 140 may transmit the blood glucose measurement data to the external device.

Meanwhile, if the first verification data and the first specific value do not match with each other and/or the second verification data and the second specific value do not match with each other, the control unit 120 may transmit flag data and the blood glucose measurement data to the external device (S1340). That is, the communication unit 140 may transmit the flag data and the blood glucose measurement data together to the external device. Herein, the flag data may be data indicating the occurrence of an error in the blood glucose measurement data.

Although steps S1310 and S1320 are sequentially displayed in FIG. 13, this is merely an example for explanation and the present invention is not limited thereto. Only any one of steps S1310 and S1320 may be performed, step S1320 may be performed before step S1310, or steps S1310 and S1320 may be performed simultaneously.

Although the above description focuses on the embodiments, these are merely examples and do not limit the present invention. Any person who has an ordinary knowledge in the field to which the present invention pertains will recognize that various modifications and applications not exemplified above are possible within the scope not departing from the essential characteristics of the present invention. For example, each component specifically shown in the embodiment can be modified and implemented. In addition, the differences related to such modifications and applications should be interpreted as being included in the scope of the present invention defined in the appended claims.

### [Description of symbols]

10: Blood glucose measurement device
30: User terminal
110: Measurement unit
120: Control unit
130: Storage unit
140: Communication unit

## Claims

1. A blood glucose measurement device comprising:
a measurement unit that is partially inserted into the body of a subject and generates a blood glucose measurement signal by measuring a blood glucose concentration of the subject;
a control unit that generates blood glucose measurement data by converting the blood glucose measurement signal into digital data; and
a storage unit that stores the blood glucose measurement data,
wherein the control unit stores preset first verification data in the storage unit, and stores the blood glucose measurement data in the storage unit continuously to the first verification data.

2. The blood glucose measurement device according to claim 1,
wherein if a preset predetermined condition is satisfied, the control unit stores second verification data continuously to the blood glucose measurement data.

3. The blood glucose measurement device according to claim 2,
wherein if storage of the blood glucose measurement data is completed without the occurrence of a specific event during the storage of the blood glucose measurement data, the control unit stores the second verification data continuously to the blood glucose measurement data.

4. The blood glucose measurement device according to claim 2,
wherein if storage of the blood glucose measurement data is stopped due to the occurrence of a specific event during the storage of the blood glucose measurement data, the control unit stores dummy data by the file size corresponding to the remaining portion of the blood glucose measurement data and the file size of the second verification data from the position where transmission was stopped.

5. The blood glucose measurement device according to claim 2,
further comprising a communication unit that transmits the stored blood glucose measurement data based on the control of the control unit.

6. The blood glucose measurement device according to claim 5,
wherein the control unit compares at least one of the first verification data and the second verification data with a specific value corresponding to each of them;
if at least one of the first verification data and the second verification data and the specific value corresponding to each of them match each other, transmits the blood glucose measurement data to an external device through the communication unit; and
if at least one of the first verification data and the second verification data and the specific value corresponding to each of them do not match each other, transmits flag data and the blood glucose measurement data to the external device through the communication unit,
wherein the flag data is data indicating the occurrence of an error for the blood glucose measurement data.

7. The blood glucose measurement device according to claim 2,
wherein at least one of the first verification data and the second verification data has a file size of at least 1 byte.

8. The blood glucose measurement device according to claim 2,
wherein at least one of the first verification data and the second verification data includes at least one of a CRC value or a parity bit.

9. A method for managing blood glucose measurement data using a blood glucose measurement device that continuously measures blood glucose of a subject,
the method comprising the steps of:
generating a blood glucose measurement signal by measuring blood glucose of the subj ect;
converting the blood glucose measurement signal into blood glucose measurement data that is a digital signal; and
storing the blood glucose measurement data,
wherein the step of storing the blood glucose measurement data includes storing preset first verification data; and
storing the blood glucose measurement data continuously to the first verification data.

10. The method for managing blood glucose measurement data according to claim 9,
wherein the step of storing the blood glucose measurement data further includes storing second verification data continuously to the blood glucose measurement data if a preset predetermined condition is satisfied.

11. The method for managing blood glucose measurement data according to claim 10,
wherein the step of storing the second verification data includes storing the second verification data continuously to the blood glucose measurement data if the storage of the blood glucose measurement data is completed without the occurrence of a specific event during the storage of the blood glucose measurement data.

12. The method for managing blood glucose measurement data according to claim 11,
wherein the step of storing the second verification data includes storing dummy data by the file size corresponding to the remaining portion of the blood glucose measurement data and the file size of the second verification data from the position where transmission was stopped if the storage of the blood glucose measurement data is stopped due to the occurrence of a specific event during the storage of the blood glucose measurement data.

13. The method for managing blood glucose measurement data according to claim 10, further comprising a step of transmitting the stored blood glucose measurement data.

14. The method for managing blood glucose measurement data according to claim 13,
wherein the step of transmitting the blood glucose measurement data includes the steps of:
comparing at least one of the first verification data and the second verification data with a specific value corresponding to each of them;
transmitting the blood glucose measurement data to an external device if at least one of the first verification data and the second verification data and the specific value corresponding to each of them match each other; and
transmitting flag data and the blood glucose measurement data to the external device if at least one of the first verification data and the second verification data and the specific value corresponding to each of them do not match each other,
wherein the flag data is data indicating the occurrence of an error for the blood glucose measurement data.

15. The method for managing blood glucose measurement data according to claim 10,
wherein at least one of the first verification data and the second verification data has a file size of at least 1 byte.

16. The method for managing blood glucose measurement data according to claim 10,
wherein at least one of the first verification data and the second verification data includes at least one of a CRC value or a parity bit.
